(19) Europäisches Patentamt
European Patent Office
Office européen des brevets

(11) Numéro de publication : **0 012 390**
**B1**

(12) # FASCICULE DE BREVET EUROPÉEN

(45) Date de publication du fascicule du brevet : **24.11.82**

(21) Numéro de dépôt : **79105000.8**

(22) Date de dépôt : **07.12.79**

(51) Int. Cl.³ : **C 07 C 45/57**, C 07 C 45/62, C 07 C 49/385, C 07 D317/26, C 07 D317/20, C 07 D493/08

(54) **Procédé pour la préparation de la muscone et composés pyranniques intermédiaires.**

(30) Priorité : **07.12.78 CH 12510/78**

(43) Date de publication de la demande : **25.06.80 (Bulletin 80/13)**

(45) Mention de la délivrance du brevet : **24.11.82 Bulletin 82/47**

(84) Etats contractants désignés : **CH DE FR GB IT NL SE**

(56) Documents cités :
**CHEMICAL ABSTRACTS, vol. 76, 1972 page 440, abrégé no. 153469d Columbus, Ohio, US S.Z. TAITS et al. : « New method for synthesizing macrocyclic compounds. XIV ; Intramolecular acylation of w-(3(or 4)-methyl-2-thienyl) alkanoic acid chlorides. Synthesis of muscone »**
**TETRAHEDRON, vol. 18, octobre 1962 Oxford GB A.T. BALABAN et al. : « Pyrylium Salts obtained by diacylation of olefins-VIII1 Intramolecular diacylation », pages 1 079-1 081.**

(73) Titulaire : **FIRMENICH SA**
**Case postale 239**
**CH-1211 Genève 8 (CH)**

(72) Inventeur : **Schulte-Elte, Karl-Heinrich**
**44, ch. de Carabot**
**CH-1213 Onex/Ge (CH)**
Inventeur : **Hauser, Arnold**
**7, ch. de la Bâtie**
**CH-1213 Petit-Lancy/Ge (CH)**
Inventeur : **Ohloff, Günther**
**13, ch. de la Chapelle**
**CH-1233 Bernex/Ge (CH)**

(74) Mandataire : **Schmied-Kowarzik, Volker, Dr. et al.**
**Patentanwälte Dipl.-Ing. P. Wirth Dr. V. Schmied-Kowarzik Dipl.-Ing. G. Dannenberg Dr. P. Weinhold Dr. D. Gudel Dipl.-Ing. S. Schubert**
**Siegfriedstrasse 8 D-8000 München 40 (DE)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

# Procédé pour la préparation de la muscone et composés pyranniques intermédiaires

La muscone, ou 3-méthyl-cyclopentadécanone, est dans la série des dérivés musqués macrocycliques, l'un parmi les ingrédients les plus appréciés dans l'industrie de la parfumerie.

Cependant, faute de synthèse économique, son utilisation a été à ce jour fort limitée.

Parmi les procédés de fabrication connus figurent ceux illustrés à l'aide des schémas réactionnels suivants :

(1.)

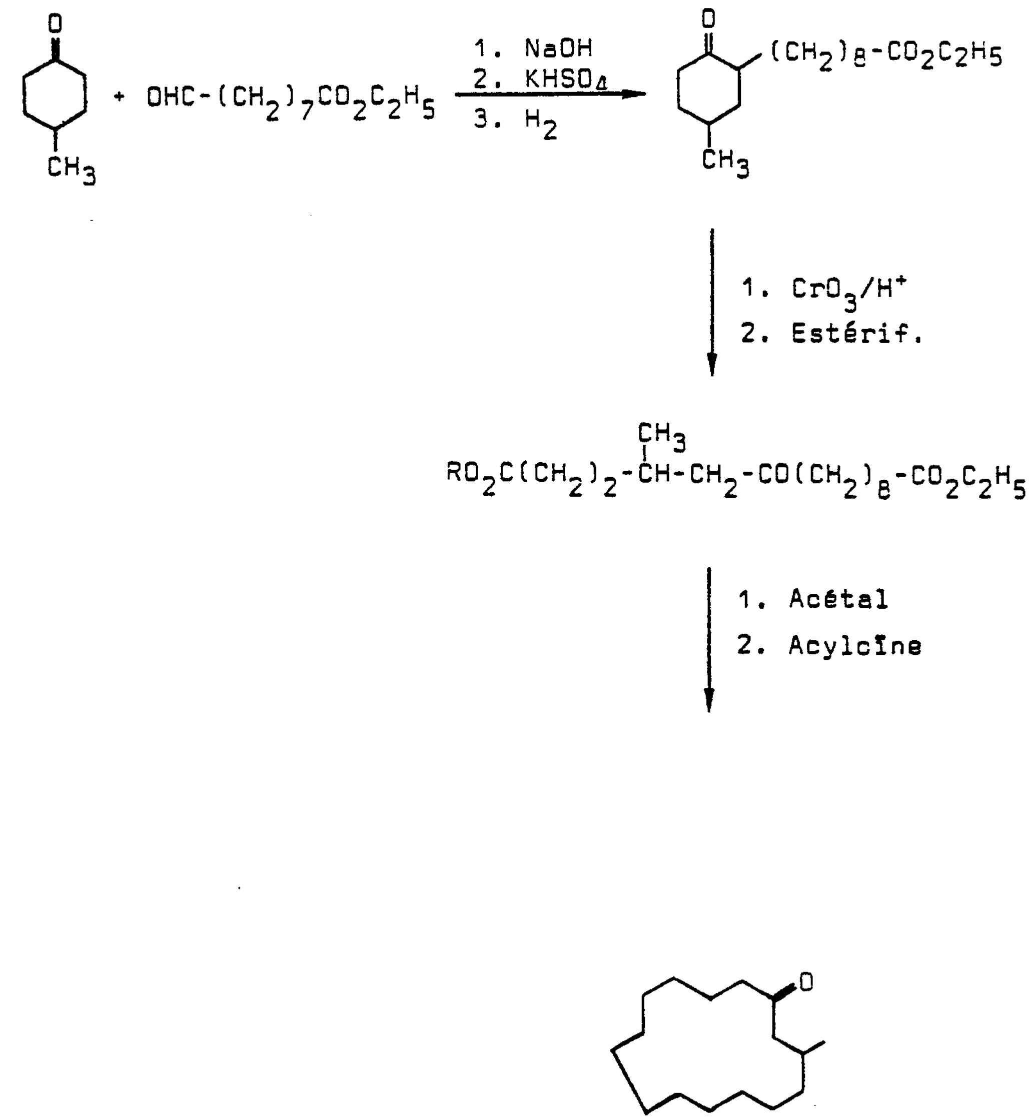


(muscone)

référence : J. Chem. Soc. 4154-7 (1964)

2.

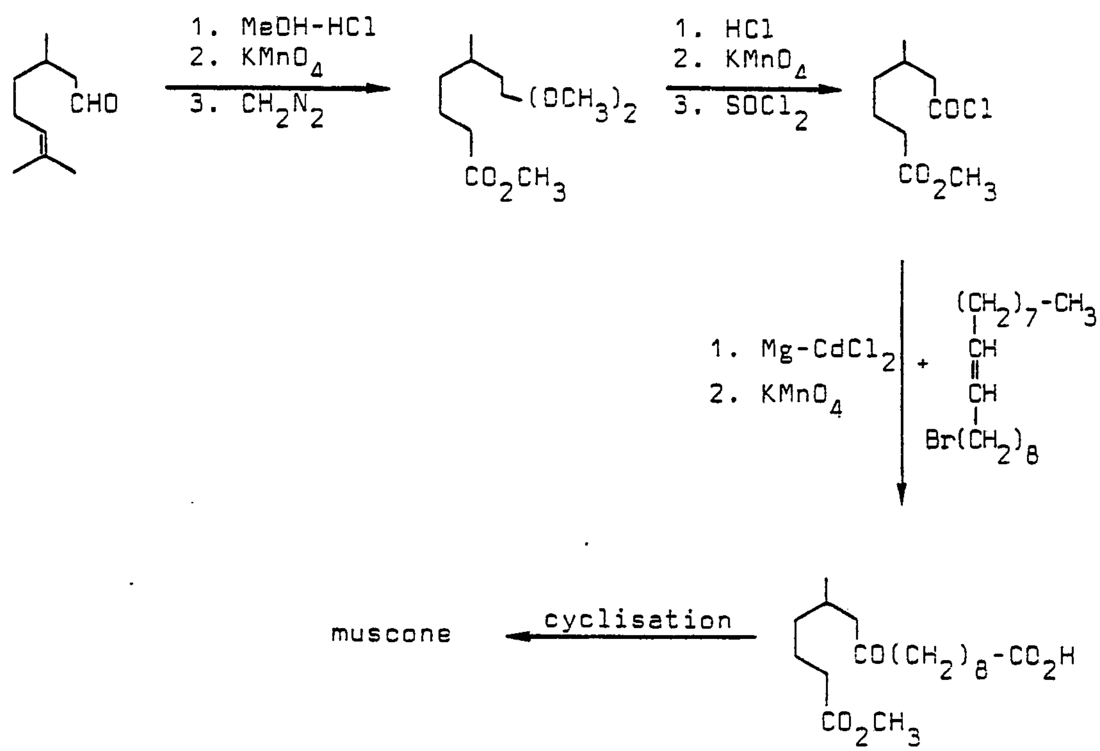


référence : Tetrahedron *20*, 11, 2601 (1964)

3.

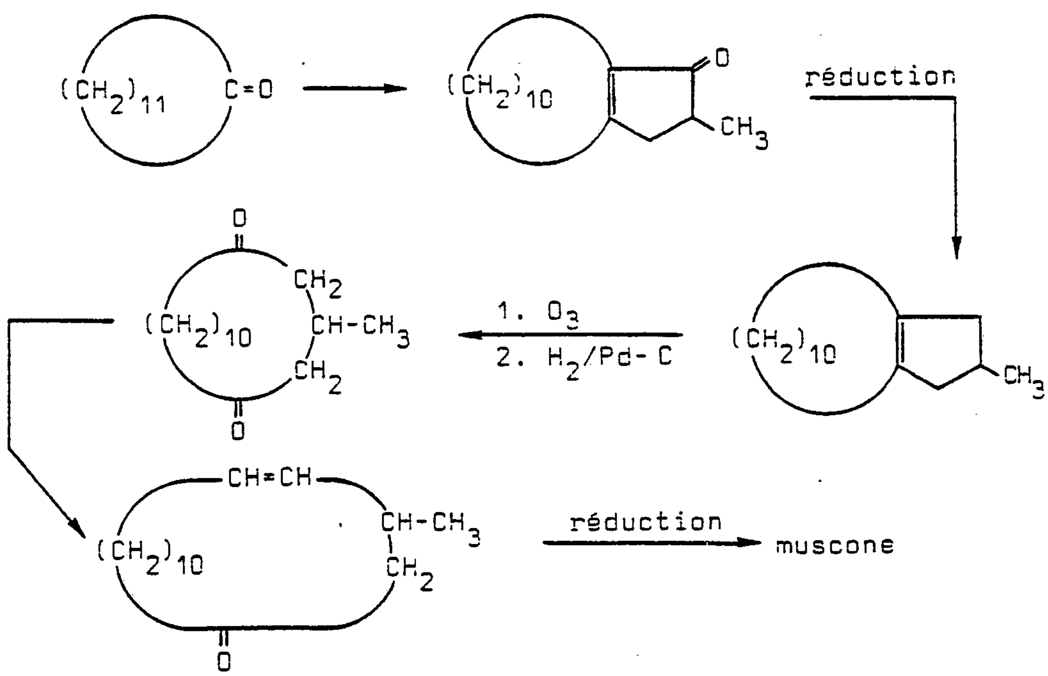


référence : Helv. Chim. Acta, *50*, 705 (1967) ou la variante ci-après :

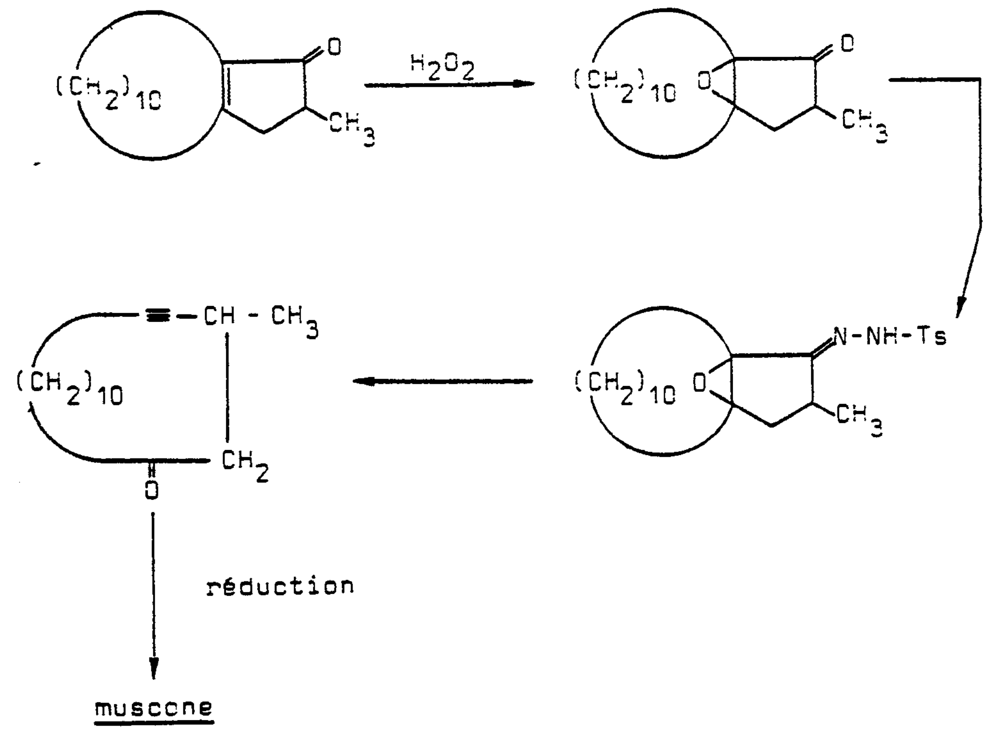


référence : Helv. Chim. Acta, *50*, 708 (1967)

« Une approche synthétique originale a également été décrite par Taits et al. [Chem. Abstr. *76*, (1972) 153-469 d] qui ont pu préparer de la muscone à partir de 3-méthylthiophène à travers la formation d'un composé de formule

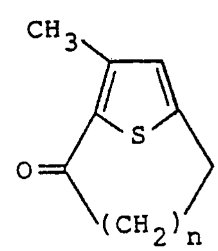

lequel a été ensuite désulfurisé à l'aide de nickel et hydrogéné en présence de palladium ».

La présente invention a pour objet un nouveau procédé pour la préparation de la muscone. Ce procédé, qui consiste en un nombre limité d'étapes de réaction, permet, grâce notamment à l'utilisation d'un produit de départ aisément accessible sur le marché, une obtention économique et à grande échelle de la muscone.

Le procédé de l'invention est caractérisé en ce qu'on

a) soumet à acétalysation un dialdéhyde de formule

$$OHC-(CH_2)_2-\overset{4}{C}(H)_m \cdots C(H)_m-(CH_2)_2-\overset{8}{C}(H)_m \cdots C(H)_m-(CH_2)_2-CHO \qquad (I)$$

contenant deux liaisons simples ou doubles dans les positions 4 et 8 comme indiqué par les pointillés, et dans laquelle l'indice m vaut 1 ou 2, pour fournir un monoacétal de formule

$$OHC-(CH_2)_2-\overset{4}{C}(H)_m \cdots C(H)_m-(CH_2)_2-\overset{8}{C}(H)_m \cdots C(H)_m-(CH_2)_2-CH\begin{matrix}OR^1 \\ OR^2\end{matrix} \qquad (II)$$

dans laquelle chacun des symboles $R^1$ et $R^2$ représente, pris séparément, un radical alkyle inférieur, ou pris conjointement comme indiqué par les pointillés, un radical alkylène inférieur,

b) additionne un halogénure de méthallyl-magnésium sur le monoacétal obtenu pour fournir un composé hydroxylé de formule

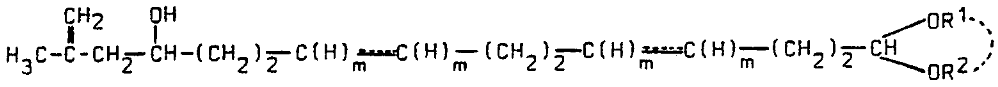

(III)

dans laquelle les symboles $R^1$ et $R^2$, l'indice m et les pointillés ont le sens indiqué ci-dessus,

c) cyclise, au moyen d'un agent de cyclisation acide, le composé de formule (III) pour obtenir le composé oxygéné bicyclique de formule

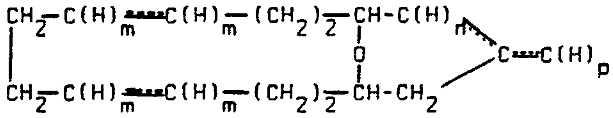

(IV)

dans laquelle l'indice m et les pointillés ont le sens indiqué plus haut et les indices n et p peuvent prendre l'une des valeurs indiquées ci-après :

a) n = 1 ; p = 3 ou
b) n = 2 ; p = 2

et

d) soumet ledit composé (IV) à une isomérisation et à une hydrogénation catalytique dans un solvant organique inerte pour fournir la muscone désirée.

Le procédé de l'invention peut être illustré à l'aide du schéma réactionnel suivant :

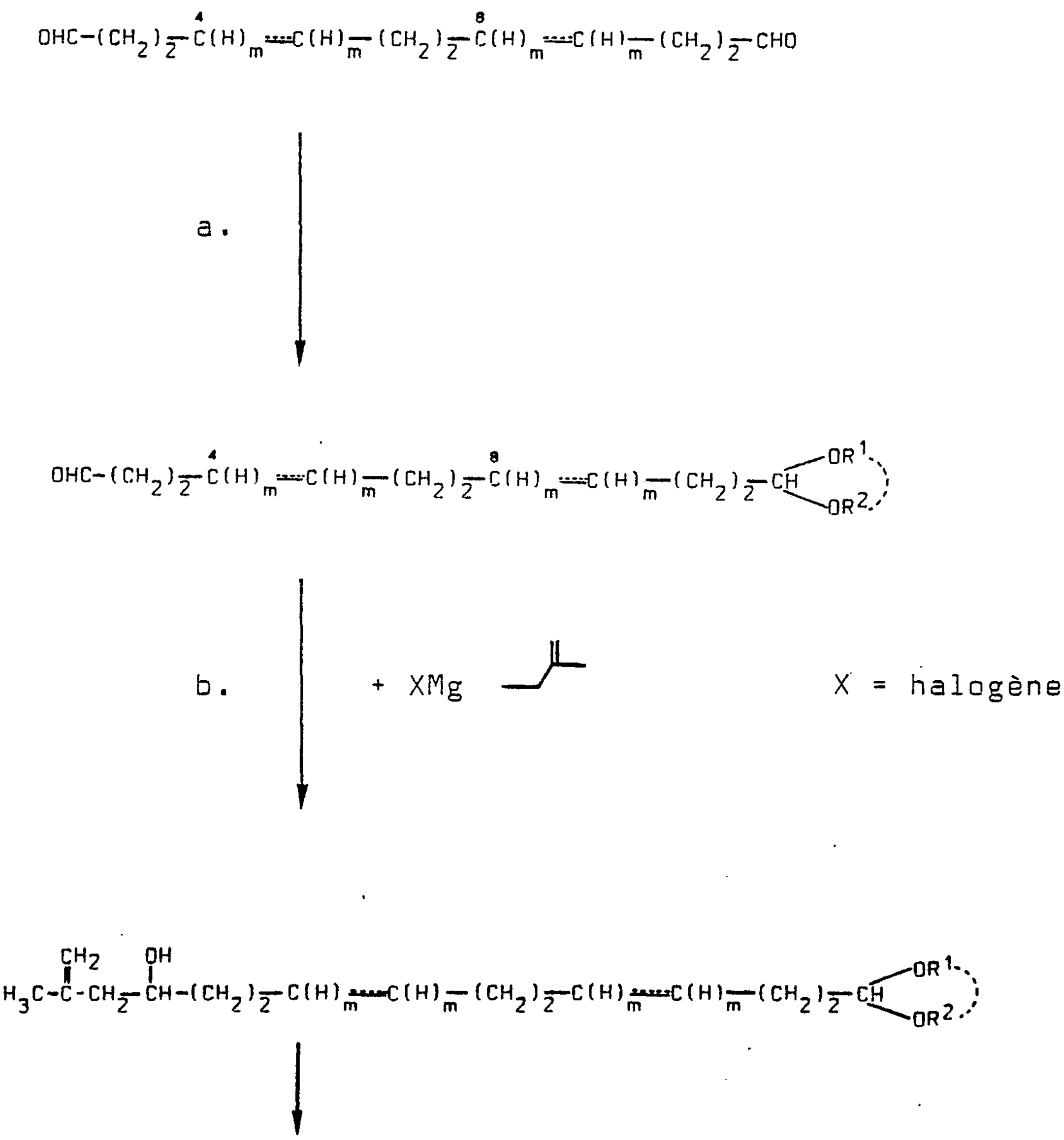


(I)

(II)

(III)

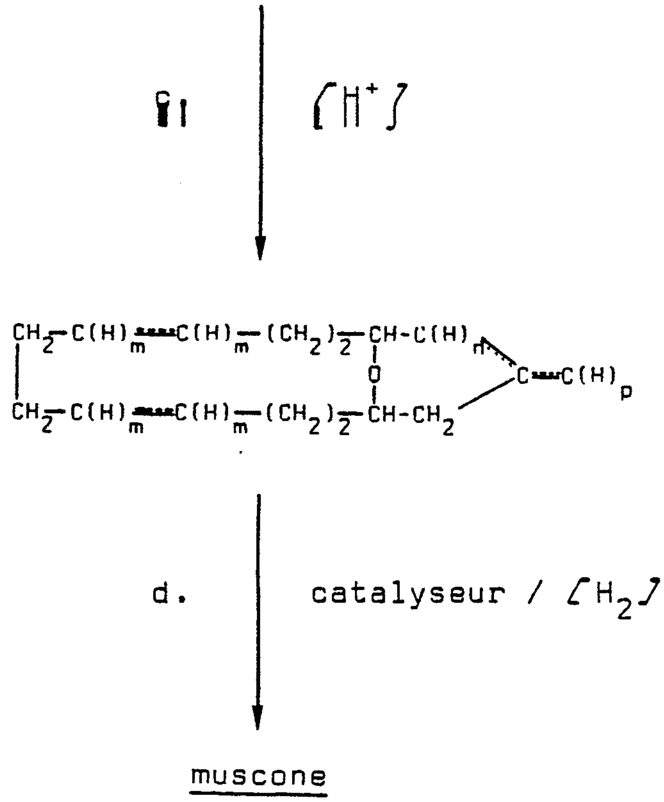


(IV)

Comme indiqué plus haut, les symboles $R^1$ et $R^2$ représentent soit des radicaux alkyles, soit des radicaux alkylènes inférieurs, préférablement avec 1-6 atomes de carbone. C'est ainsi qu'ils peuvent représenter des groupes tel le méthyle, éthyle ou propyle, ou l'éthylène, le propylène et le tétraméthylène.

Le dodéca-4,8-diène-1,12-dial (voir formule (I) dans laquelle m = 1), utilisé comme produit de départ dans le procédé de l'invention, est un composé aisément accessible à partir de cyclododécatriène au moyen d'une réaction d'ozonisation sélective d'une des doubles liaisons existantes [voir à cet effet le brevet Suisse No. 577 445].

Le dodécandial (voir formule (I) dans laquelle m = 2), également utilisé comme produit de départ dans le procédé de l'invention, peut être obtenu suivant la méthode décrite dans Compt. Rend. *204*, 1948 (1937).

A cause de la présence de doubles liaisons dans leur molécule, les composés de formule (I) à (IV) peuvent bien entendu se présenter sous différentes formes stéréo-isomériques ; le procédé de l'invention peut s'appliquer indifféremment à l'un des isomères pur ou à tout mélange desdits isomères.

La première étape du procédé de l'invention, à savoir la mono-acétalysation du dial(I), s'effectue conformément aux méthodes usuelles en présence d'un catalyseur acide, par exemple en présence d'une terre d'infusoires acide. Comme réactif préférentiel on peut utiliser l'éthylène-glycol, ce qui permet l'obtention de l'acétal éthylénique (II), dans laquelle $R^1$ et $R^2$ pris conjointement représentent un radical bivalent diméthylénique. Une fois séparé du mélange de réaction à l'aide d'une simple distillation fractionnée, le monoacétal de formule (II) a été traité avec un halogénure de méthallyl-magnésium suivi d'une hydrolyse conformément aux conditions d'une réaction de Grignard, puis le composé hydroxylé ainsi obtenu a été cyclisé à l'aide d'un agent de cyclisation acide. A cet effet des acides protoniques minéraux ou organiques ou une terre d'infusoires acide peuvent parfaitement convenir. Un tel traitement s'effectue de préférence dans un solvant organique inerte, par exemple dans un hydrocarbure aliphatique, cycloaliphatique ou aromatique ou dans un éther. Nous avons remarqué que des bons rendements en produit final étaient obtenus en effectuant la cyclisation en présence d'acide p-toluènesulfonique dans un hydrocarbure aromatique, tel le toluène, et à une température voisine de la température d'ébullition du solvant choisi, par exemple à environ 100-120 °C. Afin d'éviter la formation de produits secondaires, due en particulier à des réactions de condensation, la cyclisation est effectuée de préférence en dissolvant à grande dilution l'hydroxy-cétal dans le solvant choisi. Des concentrations de l'ordre de 1 ou 2 % en poids sont utilisées de préférence.

La dernière étape du procédé, qui consiste en l'isomérisation et l'hydrogénation catalytique du composé pyrannique de formule (IV), peut s'effectuer en présence des catalyseurs d'hydrogénation usuels. Parmi ceux-ci il convient de citer le nickel de Raney et le palladium sur charbon.

Suivant un mode préférentiel, l'étape d'isomérisation et hydrogénation s'effectue dans un solvant organique inerte, tel un hydrocarbure aromatique, comme le xylène par exemple, et à une température voisine de la température d'ébullition de celui-ci.

L'invention est illustrée d'une manière plus détaillée par les exemples suivants dans lesquels les températures sont indiquées en degrés centigrades.

Exemple 1

Préparation de la muscone

a) Dodéca-4,8-diène-1,12-dial éthylène-monoacétal

Dans un réacteur muni d'un appareil à séparateur d'eau on a ajouté goutte à goutte et avec vigoureuse agitation, 341 g (5,5 Mole) d'éthylène-glycol pendant 10-15 h à une solution maintenue à reflux de 970 g (5 Mole) de dodéca-4,8-diène-1,12-dial et 5 g de terre d'infusoires acide dans 5 l d'éther isopropylique. Une fois l'addition terminée, le mélange de réaction a été maintenu à reflux pendant 3-4 heures supplémentaires, ce qui a permis d'isoler env. 99 ml d'eau. Après filtration et évaporation des parties volatiles on a obtenu un résidu (1 200 g) qui a été ensuite soumis à une distillation fractionnée pour fournir 488 g du monoacétal désiré (pureté env. 95 %).

IR (film) : 2 700, 1 720 $cm^{-1}$ ;

RMN ($CDCl_3$, 60 MHz) : 1,5-2,6 (12H, m) ; 3,88 (4H, d, J = 2,4) : 4,84 (1H, t, J = 4) ; 5,2-5,6 (4H, m) ; 9,77 (1H, s) δ ppm ;

SM : $M^+$ = 238 ; m/e = 220 (2), 195 (15), 175 (8), 163 (2), 141 (13), 119 (14), 99 (30), 73 (100), 55 (16), 41 (42), 29 (13).

b) 2-Méthyl-4-hydroxy-pentadéca-1,7,11-triène-15-al éthylène acétal

2 ml de chlorure de méthallyle ont été ajoutés goutte à goutte sous vigoureuse agitation à une suspension de 21,4 g (0,5 Mole) de magnésium en copeaux dans 50 ml d'éther di-éthylique anhydre et 0,5 ml de bromure d'éthyle. A cette suspension on a ensuite ajouté pendant environ 3 h une solution de 119 g (0,5 Mole) du monoacétal obtenu sous lettre a. ci-dessus et 90 g (1,0 Mole) de chlorure de méthallyle (fraîchement distillé) dans 1 l d'éther anhydre et 250 ml de tétrahydrofuranne. La vitesse d'addition a été réglée de façon telle que la température ne dépasse pas 10-15° ; à cet effet un refroidissement extérieur a été nécessaire. Le mélange de réaction a été ensuite maintenu sous agitation à env. 15-20° pendant 3 h, puis la solution claire obtenue après décantation du magnésium en excès a été hydrolysée à l'aide d'une solution aqueuse saturée de $NH_4Cl$ et glace. La phase aqueuse a été extraite deux fois à l'éther et les phases organiques combinées ont été lavées avec de l'eau et une solution aqueuse de NaCl jusqu'à neutralité. Après les traitements usuels de séchage et évaporation on a obtenu un produit brut (142 g ; rend. 96 %) qui par distillation à l'aide d'un appareil à boules (160-170°/0,1 Torr) a fourni 125 g de l'hydroxy-acétal désiré.

IR (film) : 3 345, 3 080, 1 642, 890 $cm^{-1}$ ;

RMN ($CDCl_3$, 60 MHz) : 1,74 (3H, s) ; 3,5-4,1 (5H, m) ; 4,7-5,0 (3H, m) ; 5,2-5,6 (4H, m) δ ppm ;

SM : ($M^+$ − 57) = 237 ; m/e : 222 (1), 208, 195 (9), 177 (3), 159 (4), 147 (7), 141 (13), 129 (12), 119 (13), 109 (18), 99 (40), 93 (39), 79 (49), 73 (100), 67 (36), 55 (39), 43 (96), 31 (83).

c) Ether bicyclique de formule (IV ; m = 1, n = 1, p = 3 ou n = 2, p = 2)

Une solution de 14,7 g (0,05 Mole) de l'hydroxy-acétal obtenu conformément à la lettre b. ci-dessus dans 300 ml de toluène a été ajoutée à une solution maintenue à reflux de 500 mg d'acide p-toluènesulfonique dans 500 ml de toluène. La réaction a été effectuée dans un appareil muni d'un séparateur d'eau. Une fois l'addition terminée, on a ajouté une nouvelle quantité d'acide p-toluène sulfonique (200 mg) et le mélange de réaction a été concentré à environ la moitié par évaporation d'une partie du toluène présent. Le mélange concentré a été lavé deux fois avec une solution aqueuse de NaOH, séché sur $Na_2SO_4$ et évaporé sous vide. On a ainsi obtenu 13,1 g d'un résidu qui par distillation dans un appareil à boules a fourni (110-120°/0,1 Torr) 8 g (rend. 69 %) de l'éther bicyclique (IV) ayant une pureté d'environ 95 %.

RMN ($CDCl_3$, 90 MHz) : 1,66 (3H, d, J = 2) ; 3,2-3,6 (1H, m) ; 3,7-4,1 (1H, m) ; 4,15-6,9 (5H, m) δ ppm ;

SM : $M^+$ = 232 (48) ; m/e : 217 (6), 203 (11), 189 (19), 175 (7), 163 (24), 147 (36), 135 (38), 121 (86), 109 (75), 95 (100), 79 (83), 67 (73), 55 (57), 41 (99).

d) Muscone

2 800 mg de palladium sur charbon à 10 % en suspension dans 50 ml de xylène à reflux ont été activés pendant 2 h à l'aide d'un courant continu d'hydrogène (débit : 40 ml/min.). A ce mélange on a ensuite ajouté 6 960 mg (0,03 Mole) de l'éther bicyclique obtenu conformément à la lettre c. ci-dessus et, tout en maintenant le courant d'hydrogène, le mélange de réaction a été maintenu à reflux pendant 7-8 h. Le cours de la réaction a été suivi à l'aide d'un contrôle au moyen d'analyse chromatographique. L'excès d'hydrogène a été éliminé grâce à un courant d'azote et le catalyseur a été filtré, puis le mélange de

réaction a été concentré. Une distillation fractionnée à l'aide d'un appareil de type micro-Fischer a fourni 5,0 g (rend. 70 %) de muscone : Eb. 25-65°/0,1 Torr.

Exemple 2

En opérant de manière identique à celle indiquée dans l'exemple décrit ci-dessus, mais en utilisant comme produit de départ l'éthylène monoacétal de dodécandial à la place de l'éthylène monoacétal de dodéca-4,8-diène-1,12-dial, on a pu obtenir la muscone avec cependant un rendement inférieur.

a) Dodécan-1,12-dial éthylène monoacétal

rend. 10-20 %
IR : 2 710, 1 725 $cm^{-1}$ ;
RMN ($CDCl_3$, 60 MHz) : 2,1-2,6 (2H, m) ; 3,6-4,1 (4H, m) ; 4,83 (1H, t, J = 4) ; 9,75 (1H, t, J = 2) δ ppm ;
SM : $M^+$ = 242 (< 1) ; m/e : 113 (< 1), 95 (< 1), 73 (32), 62 (4), 43 (13), 31 (100).

b) 2-Méthyl-4-hydroxy-pentadécan-15-al éthylène monoacétal

rend. 95 %
IR : 3 500, 3 130, 1 655, 890 $cm^{-1}$ ;
RMN ($CDCl_3$, 60 MHz) : 1,75-2,3 (6H, m) ; 2,1 (1H, m) ; 3,5-4,1 (5H, m) ; 4,7-5,0 (3H, m) δ ppm ;
SM : ($M^+-85$) = 213 (< 1) ; 200 (< 1), 180 (< 1), 163 (1), 144 (2), 124 (10), 109 (7), 89 (28), 81 (6), 73 (22), 63 (13), 56 (100), 43 (70), 31 (56).

c) Ether bicyclique de formule (IV ; m = 2 ; n = 1, p = 3 ou n = 2, p = 2)

rend. 15-20 %
RMN ($CDCl_3$, 60 MHz) : 1,1-2,2 (25H, m) ; 3,2-3,65 (1H, m) ; 3,8-4,2 (1H, m) ; 5,24 (1H, m) δ ppm ;
SM : $M^+$ = 236 (17) ; m/e : 221 (11), 207 (< 1), 194 (4), 178 (5), 163 (2), 149 (4), 135 (8), 121 (22), 109 (34), 95 (100), 81 (43), 67 (39), 55 (54), 41 (59), 29 (20).
Ledit éther peut également être obtenu, avec un rendement de 95 %, à partir de l'éther bicyclique insaturé et préparé conformément à l'exemple précédent, voir paragraphe c., par hydrogénation en présence d'un catalyseur du type dit de Lindlar.

d) La muscone, obtenue par traitement avec $H_2$ en présence de Pd sur charbon de l'éther bicyclique, obtenu comme décrit ci-dessus, a montré des caractères analytiques en tous points identiques à ceux d'un échantillon pur.

**Revendications**

1. Procédé pour la préparation de muscone, caractérisé en ce qu'on
a) soumet à acétalysation un dialdéhyde de formule

$$OHC-(CH_2)_2-\overset{4}{C}(H)_m \cdots C(H)_m-(CH_2)_2-\overset{8}{C}(H)_m \cdots C(H)_m-(CH_2)_2-CHO \qquad (I)$$

contenant deux liaisons simples ou doubles dans les positions 4 et 8 comme indiqué par les pointillés, et dans laquelle l'indice m vaut 1 ou 2, pour fournir un monoacétal de formule

$$OHC-(CH_2)_2-\overset{4}{C}(H)_m \cdots C(H)_m-(CH_2)_2-\overset{8}{C}(H)_m \cdots C(H)_m-(CH_2)_2-CH\begin{matrix} OR^1 \\ OR^2 \end{matrix} \qquad (II)$$

dans laquelle chacun des symboles $R^1$ et $R^2$ représente, pris séparément, un radical alkyle inférieur, ou pris conjointement comme indiqué par les pointillés, un radical alkylène inférieur,

b) additionne un halogénure de méthallyl-magnésium sur le monoacétal obtenu pour fournir un composé hydroxylé de formule

$$H_3C-\overset{CH_2}{\overset{\|}{C}}-CH_2-\overset{OH}{\overset{|}{C}H}-(CH_2)_2-C(H)_m \cdots C(H)_m-(CH_2)_2-C(H)_m \cdots C(H)_m-(CH_2)_2-CH\begin{matrix} OR^1 \\ OR^2 \end{matrix} \qquad (III)$$

dans laquelle les symboles $R^1$ et $R^2$, l'indice m et les pointillés ont le sens indiqué ci-dessus,

c) cyclise, au moyen d'un agent de cyclisation acide, le composé de formule (III) pour obtenir le composé oxygéné bicyclique de formule

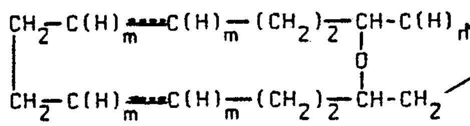

(IV)

dans laquelle l'indice m et les pointillés ont le sens indiqué plus haut et les indices n et p peuvent prendre l'une des valeurs indiquées ci-après :

a) n = 1 ; p = 3 ou
b) n = 2 ; p = 2

et

d) soumet ledit composé (IV) à une isomérisation et à une hydrogénation catalytique dans un solvant organique inerte pour fournir la muscone désirée.

2. Procédé suivant la revendication 1, caractérisé en ce que l'acétalysation du dialdéhyde (I) est effectuée au moyen d'éthylène glycol.

3. Procédé suivant la revendication 1, caractérisé en ce que la cyclisation du composé de formule (III) s'effectue au moyen d'acide p-toluènesulfonique en présence d'un solvant organique inerte et à une température de 100-120 °C.

4. Procédé suivant la revendication 1, caractérisé en ce que l'hydrogénation catalytique s'effectue en présence de palladium sur charbon en suspension dans un hydrocarbure aromatique et à une température voisine de la température d'ébullition du solvant choisi.

5. Les composés pyranniques de formule

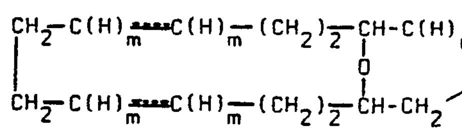

(IV)

dans laquelle les indices m, n et p ainsi que les pointillés ont le sens indiqué à la revendication 1.

6. Utilisation d'un composé pyrannique suivant la revendication 5 pour la préparation de la muscone, caractérisée en ce qu'on soumet ledit composé pyrannique à une isomérisation et à une hydrogénation catalytique dans un solvant organique inerte.

**Claims**

1. Process for the preparation of muscone, characterized in that

a) a dialdehyde of formula

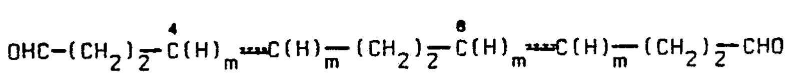

(I)

having two single or two double bonds in positions 4 and 8 as indicated by the dotted lines, and wherein index m stands for integers 1 or 2, is subjected to acetalyzation to give a monoacetal of formula

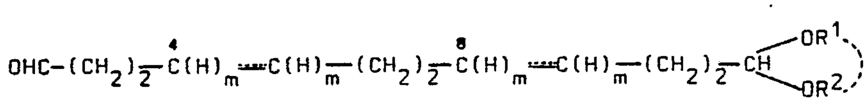

(II)

wherein each of symbols $R^1$ and $R^2$ represents, when taken separately, a lower alkyl radical or, when taken together as indicated by the dotted lines, a lower alkylene radical,

b) a methallyl-magnesium halide is added on the obtained mono-acetal to give a hydroxy compound of formula

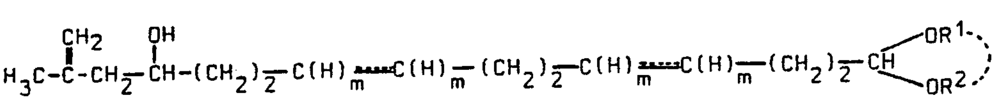

(III)

wherein symbols $R^1$ and $R^2$, index m and the dotted lines have the meaning given above,

c) the hydroxy compound of formula (III) is cyclized by means of an acidic cyclizing agent to give the oxygenated bicyclic compound of formula

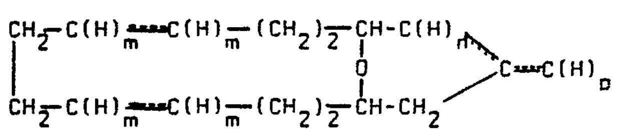

(IV)

wherein index m and the dotted lines have the meaning indicated above and indexes n and p might stand for one of the following values :

a) n = 1 ; p = 3 or
b) n = 2 ; p = 2

and

d) said compound (IV) is subjected to an isomerization and a catalytic hydrogenation in an inert organic solvent to give the desired muscone.

2. Process according to claim 1, characterized in that the acetalyzation of dialdehyde (I) is effected by means of ethylene glycol.

3. Process according to claim 1, characterized in that the cyclization of compound (III) is carried out by means of p-toluene-sulfonic acid in the presence of an inert organic solvent and at a temperature of 100-120 °C.

4. Process according to claim 1, characterized in that the catalytic hydrogenation is effected in the presence of palladium on charcoal in suspension in an aromatic hydrocarbon and at a temperature in the vicinity of the boiling point of the chosen solvent.

5. The pyranic compounds of formula

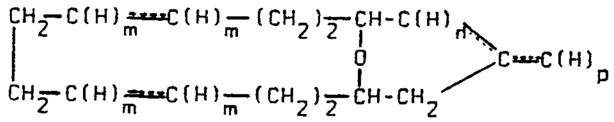

(IV)

wherein indexes m, n and p and the dotted lines have the meaning indicated in claim 1.

6. Utilization of a pyranic compound according to claim 5 for the preparation of muscone, characterized in that said pyranic compounds are subjected to an isomerization and a catalytic hydrogenation in an inert organic solvent.

**Ansprüche**

1. Verfahren zur Herstellung von Muscon, dadurch gekennzeichnet, dass

a) ein Dialdehyd der Formel

$$OHC-(CH_2)_2-\overset{4}{C}(H)_m \cdots C(H)_m-(CH_2)_2-\overset{8}{C}(H)_m \cdots C(H)_m-(CH_2)_2-CHO \quad (I)$$

mit zwei Einfach- oder zwei Doppelbindungen in Stellung 4 und 8, wie mit den gestrichelten Linien dargestellt, und worin der Index m 1 oder 2 bedeutet, acetalysiert wird, um ein Monoacetal der Formel herzustellen,

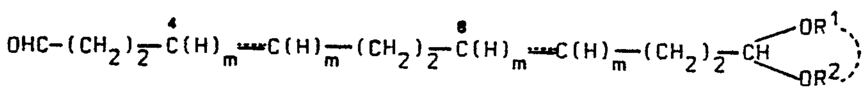

(II)

worin jedes der Symbole $R^1$ und $R^2$, getrennt genommen, einen niederen Alkylrest oder zusammen genommen, wie durch die gestrichelten Linien gekennzeichnet, einen niederen Alkylenrest darstellt,

b) ein Methallyl-Magnesium halogenid zu dem oben erhaltenen Monoacetal zugefügt wird, um eine Hydroxy-Verbindung der Formel

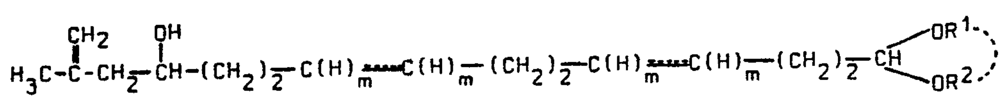

(III)

zu bilden, worin die Symbole $R^1$ und $R^2$, der Index m und die gestrichelten Linien die oben angegebene Bedeutung haben,

c) die Hydroxy-Verbindung der Formel (III) mit Hilfe eines sauren Cyclisierungsreagenz cyclisiert wird, um eine Sauerstoff enthaltende bicyclische Verbindung der Formel

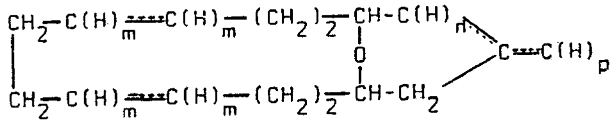

(IV)

zu bilden, wobei der Index m und die gestrichelten Linien die oben angegebene Bedeutung haben, und die Indexe n und p einen der folgenden Werte haben können :

a) $n = 1$ ; $p = 3$ oder
b) $n = 2$ ; $p = 2$,

und

d) die oben genannte Verbindung (IV) einer Isomerisierung und einer katalytischen Hydrierung in einem organischen inerten Lösungsmittel unterworfen wird, um das gewünschte Muscon zu ergeben.

2. Verfahren gemäss Anspruch 1, worin die Acetalisierung des Dialdehyds (I) mit Ethylenglykol durchgeführt wird.

3. Verfahren gemäss Anspruch 1, worin die Cyclisierung der Verbindung (III) mittels p-Toluolsulfonsäure in der Gegenwart eines inerten organischen Lösungsmittels bei einer Temperatur von 100-120 °C durchgeführt wird.

4. Verfahren gemäss Anspruch 1, worin die katalytische Hydrierung in der Gegenwart von Palladium auf Kohle in Suspension in einem aromatischen Kohlenwasserstoff und bei einer Temperatur in der Nähe des Siedepunktes des gewählten Lösungsmittels durchgeführt wird.

5. Die Pyran-Derivate der Formel

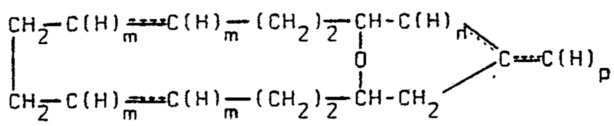

(IV)

worin die Indexe m, n und p und die gestrichelten Linien die in Anspruch 1 angegebene Bedeutung haben.

6. Verwendung eines Pyran-Derivats gemäss Anspruch 5 zur Herstellung von Muscon, dadurch gekennzeichnet, dass das genannte Pyran-Derivat einer Isomerisierung und einer katalytischen Hydrierung in einem inerten organischen Lösungsmittel unterworfen wird.